Europäisches Patentamt

(19) European Patent Office

Office européen des brevets

(11) Veröffentlichungsnummer: **0 103 177**
**B1**

(12) **EUROPÄISCHE PATENTSCHRIFT**

(45) Veröffentlichungstag der Patentschrift:
**22.04.87**

(21) Anmeldenummer: **83107959.5**

(22) Anmeldetag: **11.08.83**

(51) Int. Cl.⁴: **C 08 B 37/00**, C 12 P 19/06

(54) Abtrennung mikrobieller Polysaccharide als Amin-Addukte und deren Verwendung.

(30) Priorität: **14.08.82 DE 3230303**

(43) Veröffentlichungstag der Anmeldung:
**21.03.84 Patentblatt 84/12**

(45) Bekanntmachung des Hinweises auf die Patenterteilung:
**22.04.87 Patentblatt 87/17**

(84) Benannte Vertragsstaaten:
**AT BE CH DE FR GB IT LI LU NL SE**

(56) Entgegenhaltungen:
**US - A - 3 422 085**

(73) Patentinhaber: **HOECHST AKTIENGESELLSCHAFT,
Postfach 80 03 20, D-6230 Frankfurt am Main 80 (DE)**

(72) Erfinder: **Fischer, Edgar, Dr., Assmannshäuser Weg 8,
D-6000 Frankfurt am Main 71 (DE)**
Erfinder: **Voelskow, Hartmut, Dr., Eschenstrasse 24,
D-6234 Hattersheim am Main (DE)**

## Beschreibung

Die Erfindung betrifft ein Verfahren zur Abtrennung von mikrobiellen Polysacchariden aus ihren wässrigen Lösungen durch Fällung im sauren Medium als Addukt mit einem langkettigen Alkylamin, sowie die so erhaltenen Addukte und ihre Verwendung.

Durch Fermentation hergestellte extrazelluläre mikrobielle Polysaccharide haben aufgrund ihrer hervorragenden Eigenschaften industrielle Anwendung als Verdicker, Gelier- oder Suspendiermittel, Schutzkolloide oder wasserbindende Mittel gefunden. Bedingt durch das Herstellungsverfahren sind diese Produkte recht teuer, wobei die bisher bekannten aufwendigen Isolierverfahren erheblich zu deren hohem Preis beitragen.

Aus der US-PS 3 928 316 ist es bekannt, das anionische Heteropolysaccharid, das durch Fermentation mit Hilfe des Bakteriums Xanthomonas campestris NRRL B-1459 erhalten wurde, als wasserunlösliches Salz eines primären langkettigen Amins aus den angesäuerten verdünnten Fermentationslösungen zu isolieren. Das Fettamin wird hierbei in einer solchen Menge eingesetzt, dass alle Carboxygruppen des Polymers in Aminsalze übergeführt werden. Abgesehen von der notwendigen Verdünnung des Fermentationsmediums, die eine entsprechend aufwendige Aufarbeitung bedingt, ist das Verfahren insofern nachteilig, als das feste Fettamin notwendigerweise in Form eines wasserlöslichen Salzes eingesetzt werden muss. Arbeitet man bei Temperaturen oberhalb des Schmelzpunktes der freien Amine so wird das Xanthan mehr oder weniger stark verändert, da esterartig gebundene Acetylgruppen unter Amidbildung reagieren.

In der veröffentlichten britischen Patentanmeldung 2 053 945 ist ein ähnliches Verfahren beschrieben, bei dem zur Fällung des Polysaccharids ein Polyamin verwendet wird. Auch bei diesem Verfahren muss die Fermentationsbrühe erheblich verdünnt werden. Sofern eine Isolierung des Amins aus dem Aminsalz beabsichtigt wird, wird hier für die Behandlung des trockenen Salzes mit der Lösung einer starken Base in einer Flüssigkeit, die das freie saure Polysaccharid nicht löst wie wässriges Methanol, angegeben.

Aus der US-PS 3 119 812 ist es bekannt, das von Xanthomonas campestris NRRL B-1459 gebildete Polysaccharid mit quartären Ammoniumsalzen aus den verdünnten Fermentationsbrühen auszufällen. Hierbei ist entweder eine starke Verdünnung oder aber ein Zusatz von Alkalimetallchloriden nötig, um das Polysaccharid quantitativ zu fällen. Pro Gewichtsteil Polysaccharid werden mindestens 0,8 Gewichtsteile Ammoniumsalz eingesetzt. Die so erhaltenen Aminaddukte sind sehr stabil und erfordern zur Spaltung eine mehrfache Behandlung mit kaliumchloridhaltigem Methanol, wobei das Kaliumsalz erhalten wird. Die Herstellung des freien Xanthans erfordert eine zusätzliche Operation.

Es wurde nun ein Verfahren zur Abtrennung von mikrobiellen Polysacchariden aus ihren wässrigen Lösungen durch Fällung im sauren Medium als Addukt mit einem langkettigen Alkylamin gefunden, das dadurch gekennzeichnet ist, dass ein Amin der Formel

$$NR^1R^2R^3$$

eingesetzt wird, in der $R^1$ Alkyl mit 10 – 20 Kohlenstoffatomen und $R^2$ und $R^3$, die gleich oder verschieden sind, Methyl oder Ethyl bedeuten.

Es hat sich überraschenderweise gezeigt, dass mit den erfindungsgemäss eingesetzten tertiären Aminen keine Verdünnung der Fermentationslösungen und auch kein Salzzusatz erforderlich sind, was die Aufarbeitung erheblich vereinfacht. Vorteilhaft ist auch, dass bei Normaltemperatur flüssige Amine eingesetzt werden können, was einige bevorzugte Ausgestaltungen des Verfahrens erlaubt, die im folgenden näher beschrieben sind.

Bevorzugte tertiäre Amine für das erfindungsgemässe Verfahren sind:

Decyldimethylamin,
Dodecyldimethylamin,
Tetradecyldimethylamin,
Hexadecyldimethylamin,
Heptadecyldimethylamin,

Octadecyldimethylamin,
Undecyldimethylamin,
Eicosyldimethylamin,
Undecyldiethylamin und
Dodecylmethylethylamin.

Besonders bevorzugt sind die leicht zugänglichen Dimethylfettalkylamine, insbesondere die technischen Produkte, deren langer Alkyrest sich von Ölen und Fetten ableitet wie Kokosfett, Sojaöl oder Talgfett. Es ist unerheblich, wenn in diesen technischen Produkten geringe Anteile an Alkenylverbindungen enthalten sind. Es ist also nicht erforderlich, dass die entsprechenden Rohstoffe bzw. Amine voll durchhydriert sind. Es kommen also auch technische Rohprodukte in Betracht, was das erfindungsgemässe Verfahren zusätzlich verbilligt.

Das Verfahren ist zur Isolierung aller mikrobieller extrazellulärer Polysaccharide geeignet, die saure Gruppen aufweisen. Hierfür benötigte Mikroorganismen sind beispielsweise in der vorstehend genannten britischen Patentanmeldung 2 053 945 in der Tabelle auf den Seiten 1 – 3 aufgeführt. Von technischem Interesse sind die Heteropolysaccharide, die durch Fermentation mit Hilfe von Bakterien der Gattung Xanthomonas gebildet werden. Repräsentative Stämme hierfür sind beispielsweise X. begoniae, X. carotae, X. hederae, X. incari, X. malvacearum, X. phaseoli und insbesondere X. campestris. Das insbesondere von dem Stamm Xanthomonas campestris NRRL B-1459 gebildete Heteropolysaccharid aus Glucose-, Mannose- und Glucuronsäure-Einheiten im Polymermolekül, das noch Acetyl- und Brenztraubensäuregruppen trägt, ist unter dem Namen Xanthan bekannt. Die Herstellung dieses Produkts und ähnlicher Harze sind in der britischen Patentanmeldung 2 053 945, der darin zitierten US-PS 3 406 114 sowie in der US-PS 3 928 316 beschrieben.

Bei diesen Fermentationsverfahren wird eine Lösung mit einem Gehalt von etwa 0,5 bis 4 Gew.% Harz erhalten. Im Falle von Xanthan wählt man zweckmässig als Ausgangsmaterial Fermentationsbrühen mit einem Gehalt von etwa 1,8 bis 2 Gew.%.

In einer bevorzugten Ausgestaltung der Erfindung wird die rohe Fermentationsbrühe unmittelbar mit dem flüssigen tertiären Amin vermischt. Im allgemeinen kommt es hierbei nicht zu einer Ausfällung, was den Vorteil hat, dass man die gebildete Dispersion ohne Schwierigkeiten fördern kann und beispielsweise in ein Fällbad eintragen kann, wo das Addukt in einer für die weitere Verarbeitung günstigen Form, beispielsweise als Faden, Band, Strang oder in Form von Tropfen ausgefällt wird.

Sofern es erwünscht ist, kann bei der vorstehend genannten oder einer anderen Ausgestaltung der Erfindung das tertiäre Amin mit der Fermenterbrühe mehr oder weniger lange Zeit auf mehr oder weniger hohe Temperaturen erhitzt werden, ohne dass es zu einer Beeinträchtigung des Polymers kommt.

Die erforderliche Aminmenge kann in weiten Grenzen schwanken und ist von der Art des Polymers und den übrigen Bestandteilen der Fermenterbrühe abhängig. Die günstigste Menge kann durch einen einfachen Vorversuch leicht ermittelt werden. Im allgemeinen genügen zu einer quantitativen Abscheidung etwa 5 – 80 Gew.%, vorzugsweise 10 – 40 Gew.%, bezogen auf das Polysaccharid. Höhere Aminmengen sind ohne Nachteil möglich, erbringen jedoch auch keine Vorteile mehr.

Die Aminmenge kann besonders gering gehalten werden, wenn die aminhaltige Polymersuspension in ein saures Fällbad eingetragen wird, das einen Anteil des Amins, vorzugsweise weniger als die Hälfte, enthält.

Die Fällung des Polymers erfolgt im sauren Medium in einem pH-Bereich von etwa 2 – 6,5, vorzugsweise 3,5 bis 6,0, insbesondere 4,0 – 5,5. Zur Einstellung dieses pH-Bereichs werden vorteilhaft organische Säuren eingesetzt. Bevorzugt sind aliphatische, Monocarbonsäuren mit bis zu 5 Kohlenstoffatomen, die auch Hydroxygruppen tragen können, wie beispielsweise Ameisensäure, Essigsäure, Propionsäure, Glykolsäure oder Milchsäure. Essigsäure ist am günstigsten, da die Fällbadlösungen durch einfache Destillation regenierbar sind.

Unabhängig davon, ob die Säure zur Fällung in die Fermentationsbrühe gegeben wird oder in Form eines Fällbades vorgelegt wird, ist es vorteilhaft, zunächst das Amin der Fermenterbrühe zuzumischen, da so die erhaltenen Addukte eine sehr homogene Zusammensetzung aufweisen.

Das bei dem erfindungsgemässen Verfahren erhaltene Addukt wird in üblicher Weise, beispielsweise durch Dekantieren, Filtrieren oder Zentrifugieren, von der Mutterlauge abgetrennt und je nach Weiterverarbeitung gewaschen und beispielsweise durch Pressen weitgehend entwässert. Man kann so beispielsweise ein Produkt erhalten, das etwa 30 Gew.% Polysaccharid neben etwa 7 Gew.% Amin enthält, wobei der Rest im wesentlichen Wasser ist.

Die Verfahrensprodukte lassen sich nach bekannten Methoden, beispielsweise gemäss US-PS 3 928 316, in das Polysaccharid und das Amin zerlegen und dienen somit als Ausgangsmaterialien für die Gewinnung der freien Polysaccharide. Andererseits eignen sich die Verfahrensprodukte unmittelbar als Verdickungsmittel für die verschiedensten Einsatzzwecke. So solvatisieren die erfindungsgemäss erhaltenen Addukte – im Gegensatz zum Xanthan – in niederen Alkoholen, besonders in Methanol, unter Gelbildung und können deshalb als Verdickungsmittel für Alkohole dienen. Die Erfindung betrifft deshalb auch die Addukte selbst sowie deren Verwendung als Verdickungsmittel.

Bei vielen Anwendungen ist es vorteilhaft, die feuchten Addukte einzusetzen, da die Produkte beim Trocknen eine Änderung ihrer Eigenschaften erleiden können. Hierbei können das Solvatisierungsvermögen und die Quellbarkeit stark abnehmen.

Die feuchten Addukte quellen auch in wässrigen Lösungen vieler Salze wie Natriumchlorid, Natriumsulfat, Natriumphosphat, Kaliumchlorid, Calciumchlorid, Ammoniumchlorid, Ammoniumbromid, Ammoniumsulfat, Ammoniumcarbonat, Ammoniumformiat, Ammoniumacetat oder Ammoniumnitrat, wobei in einigen Fällen eine teilweise Lösung erfolgt. Es können auch wässrige Lösungen von Salzgemischen eingesetzt werden, beispielsweise eine Lösung mit einem Gehalt von 13 Gew.% Natriumchlorid und 1 Gew.% Calciumchlorid.

In den folgenden Beispielen beziehen sich Prozentangaben auf das Gewicht.

Die in den Beispielen eingesetzte Xanthanfermentationslösung wurde wie folgt erhalten: Als Produktionsstamm diente Xanthomonas campestris NRRL B-1459. Zur Vorkultur wurde eine Agarkultur in ein Glucose-Pepton-Medium überimpft und darin im Schüttelkolben bei 30°C bebrütet. Diese Kultur diente als Inoculum (3%) für einen 10 Liter-Fermenter, dessen Nährmedium 3–5% Glucose oder Saccharose, 0,15–0,25% Cornsteep (Maisquellwasser), 0,1 bis 0,2% Natriumnitrat, 0,1% Dikaliumphosphat und 0,05% Magnesiumsulfat-Hydrat enthielt. Der beimpfte Fermenter wurde auf 28°C gehalten und unter Rühren (400 U/min) mit 10 l Luft/min belüftet. Nach etwa 36 Stunden enthielt das Fermentationsmedium 18 – 20 g Xanthan pro Liter.

Beispiel 1

1 Liter einer Fermentationslösung mit einem Gehalt von 20 g Xanthan wurde mit 10 g Cocosfettalkyl-dimethylamin (ungefähre C-Kettenverteilung: $C_{10}$ 2%; $C_{12}$ 57%; $C_{14}$ 23%; $C_{16}$ 11%; $C_{18}$ 7%) unter Rühren versetzt. Die entstandene Dispersion wurde ebenfalls unter Rühren langsam in ein Gemisch aus 100 ml entionisiertem Wasser und 25 ml 2 N Essigsäure eingetragen. Hierbei schied sich das Addukt in Form kleiner faseriger Fladen ab, wobei die Adduktbildung zunächst im Inneren der Teilchen noch unvollständig war, aber nach Nachrühren sich vervollständigte, was am starken Schrumpfen des Niederschlags zu erkennen war. Das Addukt wurde abfiltriert, mit entionisiertem Wasser nachgewaschen und in einem Filtertuch in einer Korbpresse entwässert. Der so erhaltene Presskuchen enthielt neben 35–40 g Wasser 20 g Xanthan und etwa 5,4 g Amin. Das feuchte Produkt war in entionisiertem Wasser nicht löslich, aber teilweise löslich in einer wässrigen Lösung, die 19% Natriumchlorid und 1% Calciumchlorid enthielt, wobei das Addukt in dieser Lösung stark aufquoll.

### Beispiel 2

In 100 g einer Xanthanlösung mit einem Polysaccharidgehalt von 1,8% wurden 0,85 g Talgfettalkyl-dimethylamin (C-Kettenverteilung im Talgfettalkylrest: etwa 5% $C_{14}$; 30% $C_{16}$; 65% $C_{18}$) eingerührt. Nach Zugabe von 2,5 g 2 N Essigsäure gerann die erhaltene Dispersion und das Addukt schied sich in Form von zunächst stark gequollenen Fladen ab, die aber beim Nachrühren rasch desolvatisierten. Das Addukt wurde abfiltriert, mit entionisiertem Wasser nachgewaschen und durch Abpressen entwässert. Es wurden 6,2 g eines feuchten Presskuchens erhalten, der 1,8 g Xanthan und 0,43 g Amin enthielt.

### Beispiel 3

Beispiel 2 wurde mit der Abwandlung wiederholt, dass als Amin 0,85 g Sojafettalkyl-dimethylamin (C-Kettenverteilung im Sojaalkylrest: etwa 2% $C_{14}$; 15% $C_{16}$; 83% $C_{18}$) eingesetzt wurde. Es wurden 6,4 g eines feuchten Presskuchens erhalten, der 1,8 g Xanthan und 0,46 g Amin enthielt.

### Beispiel 4

Beispiel 2 wurde mit der Abwandlung wiederholt, dass als Amin 0,85 g Stearyl-methyl-ethylamin und anstelle der Essigsäure 3 g 2 N Milchsäure eingesetzt wurden. Man erhielt 6,2 g feuchten Presskuchen, der neben 1,8 g Xanthan 0,47 g Amin enthielt.

**Patentansprüche für die Vertragsstaaten: BE, CH, DE, FR, GB, IT, LI, LU, NL, SE**

1. Verfahren zur Abtrennung von mikrobiellen Polysacchariden aus ihren wässrigen Lösungen durch Fällung im sauren Medium als Addukt mit einem langkettigen Alkylamin, dadurch gekennzeichnet, dass ein Amin der Formel

$NR^1R^2R^3$

eingesetzt wird, in der $R^1$ Alkyl mit 10 bis 20 Kohlenstoffatomen ist und $R^2$ und $R^3$, die gleich oder verschieden sind, Methyl oder Ethyl bedeuten.

2. Verfahren nach Anspruch 1, dadurch gekennzeichnet, dass die Fällung in einem pH-Bereich von 2 – 6,5, vorzugsweise 3,5 – 6, insbesondere 4,0 – 5,5, erfolgt.

3. Verfahren nach Anspruch 1 oder 2, dadurch gekennzeichnet, dass die Fällung mit Hilfe von aliphatischen Monocarbonsäuren oder Hydroxymonocarbonsäuren erfolgt jeweils mit bis zu 5 Kohlenstoffatomen.

4. Verfahren nach einem oder mehreren der Ansprüche 1 bis 3, dadurch gekennzeichnet, dass 5 bis 80 Gew.%, vorzugsweise 10–40 Gew.% Amin, bezogen auf Polysaccharid, eingesetzt werden.

5. Verfahren nach einem oder mehreren der Ansprüche 1 bis 4, dadurch gekennzeichnet, dass das Amin der Lösung des Polysaccharids zugesetzt und anschliessend die Fällung vorgenommen wird.

6. Verfahren nach Anspruch 5, dadurch gekennzeichnet, dass die aminhaltige Polysaccharidlösung in ein saures Fällbad eingetragen wird.

7. Verfahren nach Anspruch 6, dadurch gekennzeichnet, dass ein Teil des Amins dem sauren Fällbad zugesetzt wird.

8. Addukt aus einem mikrobiellen Polysaccharid und einem Amin der Formel $NR^1R^2R^3$, in der $R^1$ Alkyl mit 10 bis 20 Kohlenstoffatomen ist und $R^2$ und $R^3$, die gleich oder verschieden sind, Methyl oder Ethyl bedeuten.

9. Verwendung des Addukts gemäss Anspruch 8 als Verdickungsmittel.

10. Verwendung des Addukts gemäss Anspruch 8 zur Herstellung der freien Polysaccharide.

**Patentansprüche für den Vertragsstaat AT**

1. Verfahren zur Abtrennung von mikrobiellen Polysacchariden aus ihren wässrigen Lösungen durch Fällung im sauren Medium als Addukt mit einem langkettigen Alkylamin, dadurch gekennzeichnet, dass ein Amin der Formel

$NR^1R^2R^3$

eingesetzt wird, in der $R^1$ Alkyl mit 10 bis 20 Kohlenstoffatomen ist und $R^2$ und $R^3$, die gleich oder verschieden sind, Methyl oder Ethyl bedeuten.

2. Verfahren nach Anspruch 1, dadurch gekennzeichnet, dass die Fällung in einem pH-Bereich von 2 – 6,5, vorzugsweise 3,5 – 6, insbesondere 4,0 – 5,5, erfolgt.

3. Verfahren nach Anspruch 1 oder 2, dadurch gekennzeichnet, dass die Fällung mit Hilfe von aliphatischen Monocarbonsäuren oder Hydroxymonocarbonsäuren erfolgt jeweils mit bis zu 5 Kohlenstoffatomen.

4. Verfahren nach einem oder mehreren der Ansprüche 1 bis 3, dadurch gekennzeichnet, dass 5 bis 80 Gew.%, vorzugsweise 10–40 Gew.% Amin, bezogen auf Polysaccharid, eingesetzt werden.

5. Verfahren nach einem oder mehreren der Ansprüche 1 bis 4, dadurch gekennzeichnet, dass das Amin der Lösung des Polysaccharids zugesetzt und anschliessend die Fällung vorgenommen wird.

6. Verfahren nach Anspruch 5, dadurch gekennzeichnet, dass die aminhaltige Polysaccharidlösung in ein saures Fällbad eingetragen wird.

7. Verfahren nach Anspruch 6, dadurch gekennzeichnet, dass ein Teil des Amins dem sauren Fällbad zugesetzt wird.

8. Verwendung des nach Anspruch 1 bis 7 erhältlichen Addukts als Verdickungsmittel.

9. Verwendung des nach Anspruch 1 bis 7 erhältlichen Addukts zur Herstellung der freien Polysaccharide.

## Claims for the Contracting states: BE, CH, DE, FR, GB, IT, LI, LU, NL, SE

1. A process for isolating microbial polysaccharides from their aqueous solutions by precipitating them in an acidic medium in the form of an adduct with a long-chain alkylamine, which comprises using an amine of the formula

$NR^1R^2R^3$

in which $R^1$ is alkyl having 10 to 20 carbon atoms, and $R^2$ and $R^3$, which are identical or different, denote methyl or ethyl.

2. The process as claimed in claim 1, wherein the precipitation is carried out within a pH range from 2 to 6.5, preferably 3.5 to 6, especially 4.0 to 5.5.

3. The process as claimed in claims 1 or 2, wherein the precipitation is carried out with the aid of aliphatic monocarboxylic acids or hydroxy-monocarboxylic acids having up to 5 carbon atoms respectively.

4. The process as claimed in any of claims 1 to 3, wherein 5 to 80% by weight preferably 10 to 40% by weight, of amine, relative to polysaccharide, are used.

5. The process as claimed in any of claims 1 to 4, wherein the amine is added to the solution of the polysaccharide and the precipitation is then carried out.

6. The process as claimed in claim 5, wherein the aminecontaining polysaccharide solution is added to an acidic coagulation bath.

7. The process as claimed in claim 6, wherein some of the amine is added to the acidic coagulation bath.

8. An adduct of a microbial polysaccharide and an amine of the formula $NR^1R^2R^3$ in which $R^1$ is alkyl having 10 to 20 carbon atoms, and $R^2$ and $R^3$, which are identical or different, denote methyl or ethyl.

9. Use of the adduct obtainable as claimed in claim 8 as thickening agent.

10. Use of the adduct as claimed in claim 8 for the production of the free polysaccharide.

## Claims for the Contracting state: AT

1. A process for isolating microbial polysaccharides from their aqueous solutions by precipitating them in an acidic medium in the form of an adduct with a long-chain alkylamine, which comprises using an amine of the formula

$NR^1R^2R^3$

in which $R^1$ is alkyl having 10 to 20 carbon atoms, and $R^2$ and $R^3$, which are identical or different, denote methyl or ethyl.

2. The process as claimed in claim 1, wherein the precipitation is carried out within a pH range from 2 to 6.5, preferably 3.5 to 6, especially 4.0 to 5.5.

3. The process as claimed in claims 1 or 2, wherein the precipitation is carried out with the aid of aliphatic monocarboxylic acids or hydroxycarboxylic acids having up to 5 carbon atoms respectively.

4. The process as claimed in any of claims 1 to 3, wherein 5 to 80% by weight preferably 10 to 40% by weight, of amine, relative to polysaccharide, are used.

5. The process as claimed in any of claims 1 to 4, wherein the amine is added to the solution of the polysaccharide and the precipitation is then carried out.

6. The process as claimed in claim 5, wherein the aminecontaining polysaccharide solution is added to an acidic coagulation bath.

7. The process as claimed in claim 6, wherein some of the amine is added to the acidic coagulation bath.

8. Use of the adduct obtainable as claimed in claims 1 to 7 as thickening agent.

9. Use of the adduct obtainable as claimed in claims 1 to 7 for the production of the free polysaccaride.

## Revendications pour les Etats contractants: BE, CH, DE, FR, GB, IT, LI, LU, NL, SE

1. Procédé pour la séparation de polysaccharides microbiens à partir de leurs solutions aqueuses, par précipitation en milieu acide sous forme d'un produit d'addition avec une alkylamine à longue chaîne, caractérisé en ce qu'on utilise une amine de formule

$NR^1R^2R^3$

dans laquelle $R^1$ représente un groupe alkyle ayant de 10 à 20 atomes de carbone, et $R^2$ et $R^3$, qui sont identiques ou différents, représentent un groupe méthyle ou éthyle.

2. Procédé selon la revendication 1, caractérisé en ce que la précipitation a lieu dans une gamme de pH de 2 à 6,5, de préférence de 3,5 à 6, en particulier de 4,0 à 5,5.

3. Procédé selon le revendication 1 ou 2, caractérisé en ce que la précipitation a lieu à l'aide d'acides monocarboxyliques aliphatiques ou d'hydroxy-acides mono-carboxyliques, ayant chacun jusqu'à 5 atomes de carbone.

4. Procédé selon une ou plusieurs des revendications 1 à 3, caractérisé en ce qu'on utilise de 5 à 80% en poids, de préférence de 10 à 40% en poids d'amine, par rapport au polysaccharide.

5. Procédé selon une ou plusieurs des revendications 1 à 4, caractérisé en ce que l'amine est ajoutée à la solution du polysaccharide et qu'on effectue ensuite la précipitation.

6. Procédé selon la revendication 5, caractérisé en ce que la solution de polysaccharide contenant une amine est introduite dans un bain de précipitation acide.

7. Procédé selon la revendication 6, caractérisé en ce qu'une partie de l'amine est ajoutée au bain de précipitation acide.

8. Produit d'addition d'un polysaccharide microbien et d'une amine de formule $NR^1R^2R^3$, dans laquelle $R^1$ représente un groupe alkyle ayant de 10 à 20 atomes de carbone et $R^2$ et $R^3$, qui sont identiques ou différents, représentent un groupe méthyle ou éthyle.

9. Utilisation du produit d'addition selon la revendication 8 en tant qu'agent épaississant.

10. Utilisation du produit d'addition selon la revendication 8 pour la préparation des polysaccharides libres.

**Revendications pour l'Etat contractant: AT**

1. Procédé pour la séparation de polysaccharides microbiens à partir de leurs solutions aqueuses par précipitation en milieu acide sous forme d'un produit d'addition avec une alkylamine à longue chaîne, caractérisé en ce qu'on utilise une amine de formule:

$NR^1R^2R^3$

dans laquelle $R^1$ représente un groupe alkyle ayant de 10 à 20 atomes de carbone, et $R^2$ et $R^3$, qui sont identiques ou différents, représentent un groupe méthyle ou éthyle.

2. Procédé selon la revendication 1, caractérisé en ce que la précipitation a lieu dans une gamme de pH de 2 à 6,5, de préférence de 3,5 à 6, en particulier de 4,0 à 5,5.

3. Procédé selon la revendication 1 ou 2, caractérisé en ce que la précipitation a lieu à l'aide d'acides monocarboxyliques aliphatiques ou d'hydroxyacides monocarboxyliques, ayant chacun jusqu'à 5 atomes de carbone.

4. Procédé selon une ou plusieurs des revendications 1 à 3, caractérisé en ce qu'on utilise de 5 à 80% en poids, de préférence de 10 à 40% en poids d'amine, par rapport au polysaccharide.

5. Procédé selon une ou plusieurs des revendications 1 à 4, caractérisé en ce que l'amine est ajoutée à la solution du polysaccharide et qu'on effectue ensuite la précipitation.

6. Procédé selon la revendication 5, caractérisé en ce que la solution de polysaccharide contenant une amine est introduite dans un bain de précipitation acide.

7. Procédé selon la revendication 6, caractérisé en ce qu'une partie de l'amine est ajoutée au bain de précipitation acide.

8. Utilisation du produit d'addition pouvant être obtenu selon l'une des revendications 1 à 7 en tant qu'agent épaississant.

9. Utilisation du produit d'addition pouvant être obtenu selon l'une des revendications 1 à 7 pour la préparation des polysaccharides libres.